(19) Europäisches Patentamt / European Patent Office / Office européen des brevets

(11) **EP 2 600 356 B1**

(12) **EUROPEAN PATENT SPECIFICATION**

(45) Date of publication and mention
of the grant of the patent:
**17.05.2017 Bulletin 2017/20**

(51) Int Cl.:
***A61N 5/10*** (2006.01)     ***G21K 5/00*** (2006.01)
***G21K 5/02*** (2006.01)

(21) Application number: **11812263.9**

(22) Date of filing: **12.07.2011**

(86) International application number:
**PCT/JP2011/065885**

(87) International publication number:
**WO 2012/014671 (02.02.2012 Gazette 2012/05)**

(54) **NEUTRON RAY IRRADIATION DEVICE, AND METHOD FOR CONTROL OF NEUTRON RAY IRRADIATION DEVICE**

NEUTRONENBESTRAHLUNGSVORRICHTUNG UND VERFAHREN ZUR STEUERUNG DER NEUTRONENBESTRAHLUNGSVORRICHTUNG

DISPOSITIF D'IRRADIATION PAR UN RAYONNEMENT DE NEUTRONS ET PROCÉDÉ DE COMMANDE DU DISPOSITIF D'IRRADIATION PAR UN RAYONNEMENT DE NEUTRONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2010 JP 2010169210**

(43) Date of publication of application:
**05.06.2013 Bulletin 2013/23**

(73) Proprietor: **Sumitomo Heavy Industries, Ltd.
Tokyo 141-6025 (JP)**

(72) Inventor: **MITSUMOTO Toshinori
Tokyo 188-8585 (JP)**

(74) Representative: **Wagner, Karl H.
Wagner & Geyer
Gewürzmühlstrasse 5
80538 München (DE)**

(56) References cited:
**FR-A1- 2 897 502          JP-A- 2004 233 168
JP-A- 2004 337 268      US-A- 4 112 306
US-A- 5 392 319          US-A1- 2001 014 134**

• **BLISS M ET AL: "Real-time dosimetry for boron neutron-capture therapy", NUCLEAR SCIENCE SYMPOSIUM AND MEDICAL IMAGING CONFERENCE, 1994., 1994 IEEE CONFERENCE RECORD NORFOLK, VA, USA 30 OCT.-5 NOV. 1994, NEW YORK, NY, USA,IEEE, US, vol. 2, 30 October 1994 (1994-10-30), pages 935-939, XP010150245, DOI: 10.1109/NSSMIC.1994.474466 ISBN: 978-0-7803-2544-9**

EP 2 600 356 B1

**Description**

Technical Field

The present invention relates to a neutron beam irradiation apparatus and a control method thereof.

Background Art

Boron neutron capture therapy (BNCT: Boron NCT) in which cancer treatment is carried out by irradiation with a neutron beam has become known as a radiation therapy in cancer treatment and the like. In the past, a neutron beam irradiation apparatus (BNCT apparatus) used to carry out the boron neutron capture therapy was developed. For example, PTL 1 discloses that a proton beam (charged particle beam) is generated by the use of an accelerator such as cyclotron, a neutron beam is generated by irradiating a target of beryllium or the like with the proton beam, and an irradiation object such as a patient is irradiated with the generated neutron beam.

Summary of Invention

Technical Problem

In the neutron beam irradiation apparatus, the dose of a neutron beam during irradiation is measured, for example, by attaching a gold wire for measuring a neutron beam to an irradiation object in advance, detaching the gold wire therefrom during the irradiation with a neutron beam, and measuring an amount of activated gold of the gold wire. It is intended to control (for example, stop) the neutron beam irradiation apparatus so as to irradiate the irradiation object with the neutron beam with a desired dose on the basis of the measured dose.

However, in this case, for example, when a dose rate of a neutron beam varies for some reasons after measuring the amount of activated gold of the gold wire, it may not be possible to cope with this variation and it may thus be difficult to irradiate an irradiation object with a neutron beam with a desired dose. Accordingly, in the neutron beam irradiation apparatus, there is a need for improvement of precision of a dose of a neutron beam with which an irradiation object is irradiated.

FR 2 897 502 A discloses a target for producing an emerging beam of neutrons from an incident beam of charged particles, the target comprising: a neutron-generator material; and an actuator system for responding to receiving a control signal by varying the thickness of neutron-generator material opposed to the incident beam.

US 5,392,319 A discloses a system and apparatus for producing neutron beams having an energy spectrum and intensity suitable for use in neutron capture therapy. The apparatus employs a rotating carriage which supports an annular target support region carrying a target material such as lithium metal. The carriage rotatably positions the target material before the pathway of charged particles generated from an accelerator course of ions. To cool the assemblage, the carriage is formed having an internally disposed chamber within which a coolant is directed from an external source. Because of the centrifugal forces imposed upon this internally-disposed coolant, it is forced to a heat exchange position adjacent the target region. The assemblage of carriage and accelerator are retained within a containment chamber under vacuum. Surrounding these components at the vicinity of the charged particle or ion pathway is a containment and moderator assembly serving to modulate the energies of generated neutrons while reflecting desired neutrons into a neutron output stream and absorbing thermal neutrons and undesired gamma radiation.

BLISS M et al.: "Real-time dosimetry for boron neutron-capture therapy", NUCLEAR SCIENCE SYMPOSIUM AND MEDICAL IMAGING CONFERENCE, 1994 IEEE CONFERENCE RECORD, Norfolk, VA, USA, 30 OCT. - 5 NOV. 1994, vol. 2, pages 935-939, discloses that in epithermal/thermal boron neutron-capture therapy (BNCT) as a treatment method for malignant tumors, because the doses and dose rates for medical therapeutic radiation are very close to the normal tissue tolerance, small errors in radiation delivery can result in harmful overdoses. A substantial need exists for a device that will monitor, in real time, the radiation dose being delivered to a patient. Pacific Northwest Laboratory (PNL) has developed a scintillating glass optical fiber that is sensitive to thermal neutrons. The small size of the fibers offers the possibility of in vivo dose monitoring at several points within the radiation field. The count rate of such detectors may approach 10 MHz because the lifetime of the cerium activator is fast. Fluxes typical of those in BNCT (i.e., $10^9$ n/cm$^2$/sec) may be measured because of this potentially high count rate and the small diameter of the fiber.

Moreover, attention is drawn to US 4,112,306 A and US 2001/014134 A1.

Therefore, an object of the invention is to provide a neutron beam irradiation apparatus which can improve precision of a dose of a neutron beam and a control method thereof.

Solution to Problem

To achieve the above-mentioned obj ect, the inventors found, as the result of active study, that it was possible to cope with the variation in dose rate of a neutron beam and thus to improve the precision of the dose of a neutron beam, when the neutron beam irradiation apparatus could grasp the dose of a neutron beam online at the time of irradiating an irradiation object with a neutron beam. Since there is a fixed correlation between the dose rate of a charged particle beam and the dose rate of a neutron beam, the inventors made the invention by finding that the dose of a neutron beam could be suitably grasped online using the correlation by sequentially measuring the dose of a charged particle beam to a target during irradiation with the neutron beam.

That is, according to an aspect of the invention, there is provided a neutron beam irradiation apparatus as set forth in claim 1.

In the neutron beam irradiation apparatus, since the dose of the charged particle beam is measured in real time during the irradiation of the neutron beam, it is possible to satisfactorily grasp the dose of a neutron beam for the above-mentioned reasons and thus to improve precision of the dose of a neutron beam.

It is preferable that the neutron beam irradiation apparatus control the irradiation of the irradiation object with the neutron beam on the basis of the dose of the charged particle beam measured by the measuring means. In this case, the irradiation with the neutron beam is controlled depending on the dose of the neutron beam grasped online.

The neutron beam irradiation apparatus includes a conversion unit that converts the dose of the charged particle beam measured by the measuring means into a dose of the neutron beam. The neutron beam irradiation apparatus may further include display means for displaying the dose of the neutron beam converted by the conversion unit. By including the display means, a doctor or an operator can grasp the dose of the neutron beam during the irradiation with the neutron beam.

According to another aspect of the invention, there is provided a control method of a neutron beam irradiation apparatus as set forth in claim 4.

In the control method of a neutron beam irradiation apparatus, since the dose of the charged particle beam is measured in real time, it is possible to satisfactorily grasp the dose of a neutron beam for the above-mentioned reasons and thus to improve precision of the dose of a neutron beam.

It is preferable that the control method of a neutron beam irradiation apparatus further comprises that the control means control the irradiation of the irradiation object with the neutron beam on the basis of the dose of the charged particle beam measured in the measuring step. In this case, the irradiation with the neutron beam is controlled depending on the dose of the neutron beam suitably grasped online.

The control method of a neutron beam irradiation apparatus includes conversion means converting the dose of the charged particle beam measured by the measuring means into a dose of the neutron beam. The control method of a neutron beam irradiation apparatus may further include display means displaying the dose of the neutron beam converted by the conversion means. By including the display means, a doctor or an operator can grasp the dose of the neutron beam during the irradiation with the neutron beam.

Advantageous Effects of Invention

According to the aspects of the invention, it is possible to improve the precision of a dose of a neutron beam.

Brief Description of Drawings

FIG. 1 is a diagram illustrating the configuration of a neutron beam irradiation apparatus according to an embodiment of the invention.
FIG. 2 is a perspective view schematically illustrating a neutron beam generating unit in the neutron beam irradiation apparatus shown in FIG. 1.
FIG. 3 is a graph illustrating the relationship between a dose rate of a charged particle beam and a dose rate of a neutron beam.
FIG. 4 is a diagram illustrating measuring means according to a modified example of the invention.

Description of Embodiments

Hereinafter, an exemplary embodiment of the invention will be described in detail with reference to the accompanying drawings. In the following description, identical or similar elements will be referenced by identical reference numerals and description thereof will not be repeated. Terms, "upstream" and "downstream", mean an upstream side (cyclotron side) and a downstream side (irradiation object side) of a charged particle beam and a neutron beam to be emitted.

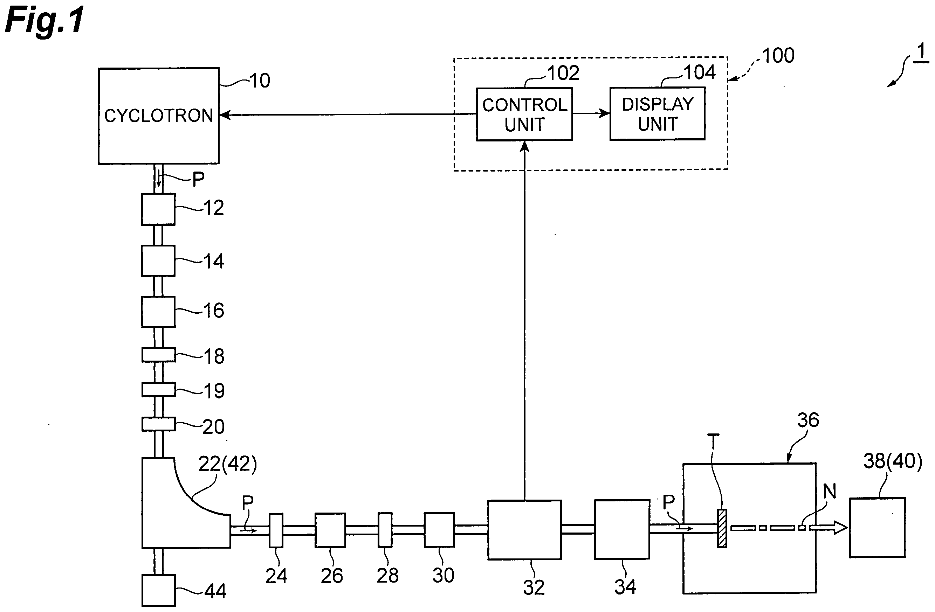

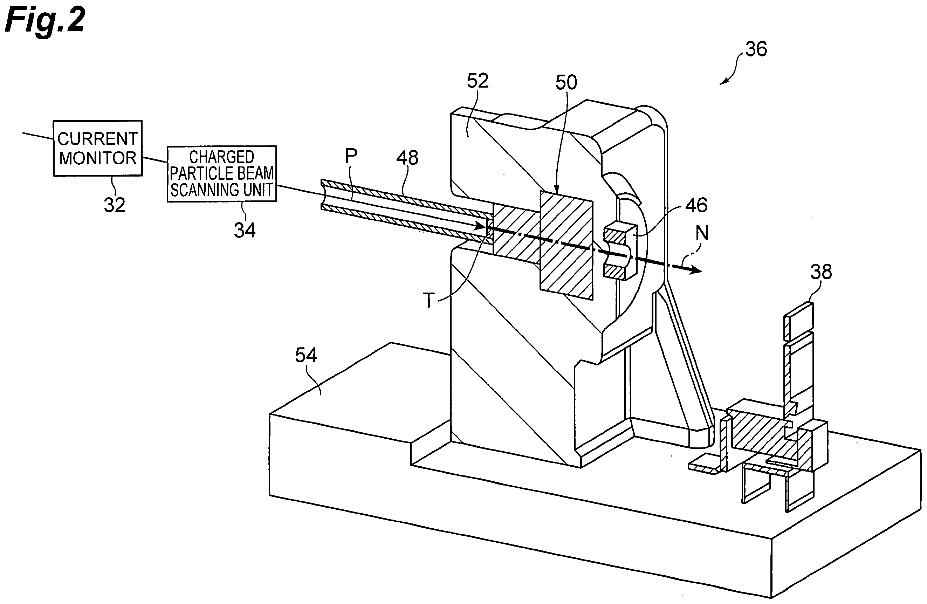

FIG. 1 is a diagram illustrating the configuration of a neutron beam irradiation apparatus according to an embodiment of the invention. FIG. 2 is a perspective view schematically illustrating a neutron beam generating unit in the neutron beam irradiation apparatus shown in FIG. 1. As shown in FIG. 1, the neutron beam irradiation apparatus 1 is an apparatus used to carry out cancer treatment or the like, for example, using a neutron capture therapy, and irradiates an irradiation object 40 such as a patient with a neutron beam N.

The neutron beam irradiation apparatus 1 includes a cyclotron 10. The cyclotron 10 accelerates charged particles such as protons to generate a proton ray (proton beam) as a charged particle beam P. Here, the cyclotron 10 has capability of generating a charged particle beam P with a beam radius of 40 mm and 60 kW (=30 MeVx2 mA).

A charged particle beam P emitted from the cyclotron 10 sequentially passes through a horizontal steering 12, a four-direction slit 14, a horizontal and vertical steering 16, quadrupole electromagnets 18, 19, and 20, a 90-degree deflecting electromagnet 22, a quadrupole electromagnet 24, a horizontal and vertical steering 26, a quadrupole electromagnet 28, a four-direction slit 30, a current monitor 32, and a charged particle beam scanning unit 34 and is guided to a neutron beam generating unit 36. The charged particle beam P is applied to a target T in the neutron beam generating unit 36 to generate a neutron beam N. The neutron beam N is applied to an irradiation object 40 on a treatment table 38.

The horizontal steering 12 and the horizontal and vertical steerings 16 and 26 serve to suppress divergence of the charged particle beam, for example, using an electromagnet. Similarly, the quadrupole electromagnets 18, 19, 20, 24, and 28 serve to adjust a beam axis of the charged particle beam P, for example, using an electromagnet. The four-direction slits 14 and 30 serve to shape the charged particle beam P by cutting beam at an end.

The 90-degree deflecting electromagnet 22 serve to deflect the traveling direction of the charged particle beam P by 90 degrees. The 90-degree deflecting electromagnet 22 includes a conversion unit 42 and can cause the charged particle beam P to depart from a regular orbit through the use of the conversion unit 42 and can guide the charged particle beam to a beam dump 44. The beam dump 44 is used to check the output of the charged particle beam P before treatment or the like.

The current monitor 32 measures a current value (that is, an amount of charge, a dose rate) of a charged particle beam P with which a target T is irradiated in real time. The current monitor 32 employs a non-destructive DCCT (DC Current Transformer) which can measure current without affecting the charged particle beam P. The controller 100 to be described later is connected to the current monitor 32. The "dose rate" means a dose per unit time (the same is true of the following).

The charged particle beam scanning unit 34 scans the target T with the charged particle beam P to control the irradiation of the target T with the charged particle beam P. Here, the charged particle beam scanning unit 34 controls the irradiation position of the target T with the charged particle beam P, the beam diameter of the charged particle beam P, and the like.

As shown in FIG. 2, the neutron beam generating unit 36 generates a neutron beam N by irradiating the target T with the charged particle beam P and emits the generated neutron beam N through a collimator 46. The neutron beam generating unit 36 includes a target T disposed at a downstream end of a beam duct 48 through which the charged particle beam P passes, a decelerating member 50 that decelerates the neutron beam N generated from the target T, and a shielding member 52 that is disposed to cover these elements.

The target T is irradiated with the charged particle beam P to generate a neutron beam N. Here, the target T is formed of, for example, beryllium (Be) and has a disk shape with a diameter of 160 mm. The decelerating member 50 serves to decelerate the energy of the neutron beam N and has, for example, a multi-layered structure including plural different materials. The shielding member 52 blocks the generated neutron beam N, gamma rays generated with the generation of the neutron beam N, and the like so as not to be emitted to the outside and is disposed on a floor 54.

Referring to FIG. 1 again, the neutron beam irradiation apparatus 1 includes the current monitor 32 that can measure the current value of the charged particle beam P in real time as described above, and the current monitor 32 is connected to the controller 100.

The current monitor 32 is disposed between the cyclotron 10 and the neutron beam generating unit 36 in the beam path of the charged particle beam P. Specifically, the current monitor 32 is disposed between the 90-degree deflecting electromagnet 22 and the neutron beam generating unit 36 in the beam path of the charged particle beam P so as to accurately measure the dose of the charged particle beam P with which the target T is irradiated and to exclude the adverse influence of the 90-degree deflecting electromagnet 22. Particularly, the current monitor 32 according to this embodiment is preferably disposed on the downstream side (that is, on the neutron beam generating unit 36 side) of the beam path of the charged particle beam P and just before the charged particle beam scanning unit 34. Since the charged particle beam scanning unit 34 scans the target T with the charged particle beam P, a large-sized current monitor 32 is required for disposing the current monitor 32 downstream from the charged particle beam scanning unit 34. On the contrary, by disposing the current monitor 32 upstream from the charged particle beam scanning unit 34, it is possible to reduce the size of the current monitor 32.

The controller 100 includes a control unit 102 and a display unit 104. The control unit 102 serves to calculate the dose of the neutron beam N from the current value of the charged particle beam P measured by the current monitor 32 and to control the irradiation with the neutron beam N on the basis of the dose, and includes, for example, a CPU, a ROM, and a RAM. The display unit 104 serves to display the dose of the neutron beam N calculated by the control unit 102 and employs, for example, a display device or a monitor.

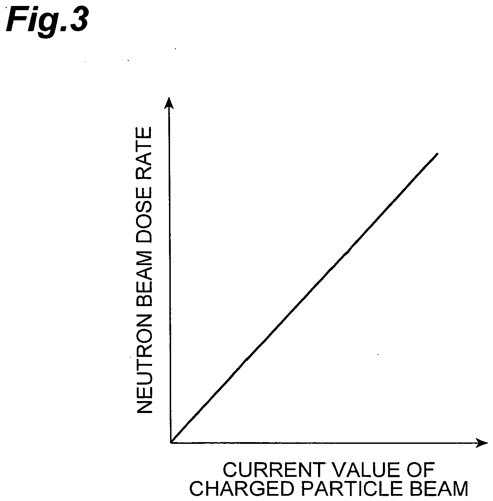

Here, in the neutron beam irradiation apparatus 1 according to this embodiment, the following control (control method) is carried out using the proportional relationship (see FIG. 3) between the current value of the charged particle beam P and the dose rate of the neutron beam N through the use of the current monitor 32 and the controller 100.

That is, the target T is irradiated with the charged particle beam P emitted from the cyclotron 10 and the current value of the charged particle beam P is measured in real time by the current monitor 32 while an irradiation object 40 is being irradiated with the neutron beam N generated from the target T. In addition, the measured current values of the charged particle beam P are sequentially integrated with respect to the time by the control unit 102 and the dose of the charged particle beam P is measured in real time. The dose of the charged particle beam P is converted into the dose of the neutron beam N using Expression 1. That is, the dose of the neutron beam N is calculated from the dose of the charged particle beam P. Accordingly, the dose is measured online during the irradiation with the neutron beam N.

Expression 1

$$\text{Dose of Neutron Beam} \propto \int I(t) dt$$

Here, I represents the current value of a charged particle beam.

Subsequently, the resultant dose of the neutron beam N is displayed on the display unit 104 to inform an operator such as a doctor of the dose of the neutron beam N. When the calculated dose of the neutron beam N reaches a planned value (a predetermined dose planned for irradiation), the function of the cyclotron 10 is stopped by the control unit 102 to stop the generation of the charged particle beam P, thereby stopping the generation of the neutron beam N and the irradiation of the irradiation object 40.

In this embodiment, by measuring the dose of the charged particle beam P in real time before irradiating the target T instead of directly measuring the neutron beam N, it is possible to appropriately measure and grasp the dose of the neutron beam N online (in real time). As a result, for example, when the dose rate varies during the irradiation with the neutron beam N, it is possible to cope with this variation and thus to satisfactorily and accurately irradiate the irradiation object 40 with the neutron beam N with a dose corresponding to a planned value. That is, by extending the irradiation time when it is detected that the dose rate is lowered during the irradiation with the neutron beam N and shortening the irradiation time when it is detected that the dose rate is raised during the irradiation with the neutron beam N, it is possible to satisfactorily and accurately irradiate the irradiation object with the neutron beam N with a dose corresponding to a planned value.

In this way, in this embodiment since the dose of the neutron beam N can be grasped online, it is not necessary to temporarily stop the irradiation with the neutron beam N to grasp the dose of the neutron beam N unlike the related art, and it is possible to suppress the re-irradiation with the neutron beam due to the lack dose of the neutron beam N. Therefore, according to this embodiment, it is possible to shorten the treatment time (the time from the start of irradiation to the end thereof) and thus to improve an apparatus operation rate.

In this embodiment, the function of the cyclotron 10 is stopped by the control unit 102, but a shutter or the like disposed in the beam path of the charged particle beam P is activated to block the irradiation with the charged particle beam P, as long as the irradiation with the neutron beam N can be stopped. The control unit 102 may control the dose rate of the neutron beam N to raise or lower the dose rate of the neutron beam N depending on the dose of the neutron beam N in addition to the online-measured dose of the charged particle beam P, as well as controlling the stopping of the irradiation with the neutron beam N. That is, the control unit 102 has only to control the irradiation of the irradiation object 40 with the neutron beam N on the basis of the dose of the charged particle beam P.

In this embodiment, the proportional relationship (linear relationship) between the current value of the charged particle beam P and the dose rate of the neutron beam N may be calibrated in advance, by preferably causing the charged particle beam scanning unit 34 to scan the target with the charged particle beam P, for example, so as to cause the charged particle beam P to satisfactorily collide with the target T before actually irradiating the irradiation object 40 with the neutron beam N.

In this embodiment, the cyclotron 10 constitutes the charged particle beam generating means, and the neutron beam generating unit 36 constitutes the neutron beam generating means. The current monitor 32 and the control unit 102 constitute the measuring means, the control unit 102 constitutes the control means and the conversion unit, and the display unit 104 constitutes the display means.

While an exemplary embodiment of the invention has been described, the invention is not limited to the embodiment and may be modified or applied to another configuration without departing from the scope of the appended claims.

For example, the material of the target T is not limited to beryllium, but may be tantalum (Ta) or tungsten (W), or the like. In the above-mentioned embodiment, the current value of the charged particle beam P is measured in real time by the use of the current monitor 32, but the invention is not limited to this configuration. For example, the current value of the charged particle beam P may be measured in real time as follows.

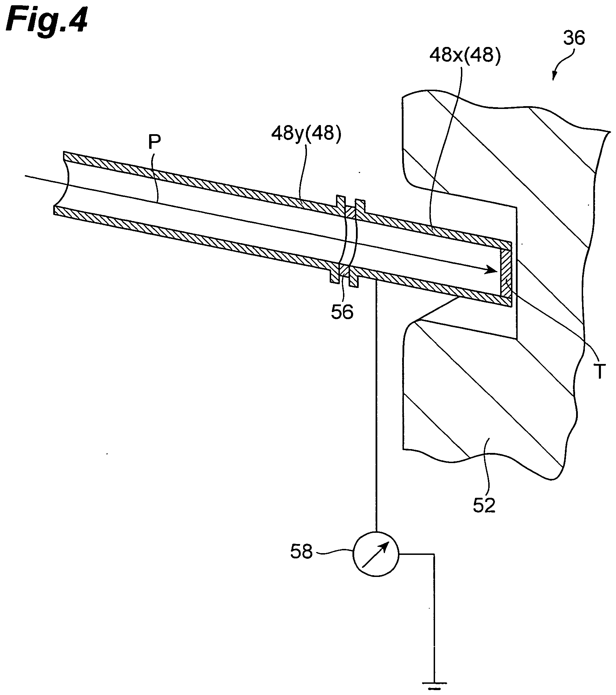

As shown in FIG. 4, in a neutron beam irradiation apparatus according to a modified example, the beam duct 48 (see FIG. 2) connected to the neutron beam generating unit 36 is divided into a downstream (target T side) beam duct 48x and an upstream beam duct 48y. An annular spacer 56 formed of an insulator such as ceramic is interposed between the beam ducts 48x and 48y, and thus the downstream beam duct 48x is electrically insulated from the other parts. In this state, by measuring current, which is generated from the target T by irradiating the target T with the charged particle beam P and which flows from the target T to the downstream beam duct 48x, by the use of an ammeter 58, the dose of the charged particle beam P with which the target T is irradiated may be measured in real time. The relationship between the current flowing in the downstream beam duct 48x and the dose of the charged particle beam P with which the target T is irradiated is calculated in advance.

In the above-mentioned embodiment, the dose of the charged particle beam P is measured in real time by integrating the current value of the charged particle beam P with respect to the time, and the dose of the charged particle beam P is converted into the dose of the neutron beam N through the use of Expression 1, but the invention is not limited to this configuration. For example, by converting the current value of the charged particle beam P into the dose rate of the neutron beam N and integrating the dose rate of the neutron beam N with respect to the time, the dose of the charged particle beam P may be calculated. In this case, the current value of the charged particle beam P is integrated in practice and thus the dose of the charged particle beam P is measured in real time.

In the above-mentioned embodiment, charged particles are accelerated using the cyclotron 10, but other accelerators such as a synchrotron, synchrocyclotron, and a linear accelerator other than the cyclotron may be employed.

In the above-mentioned embodiment, the charged particle beam P is deflected by 90 degrees using the 90-degree deflecting electromagnet 22, but the elements from the cyclotron 10 to the neutron beam generating unit 36 in the beam path of the charged particle beam P may be arranged in a line shape without using the 90-degree deflecting electromagnet.

In the above-mentioned embodiment, the irradiation with the neutron beam N is automatically stopped by the control unit 102 when the calculated dose of the neutron beam N reaches a planned value, but an operator such as a doctor may check the dose of the neutron beam N displayed on the display unit 104 and the operator may manually stop the irradiation with the neutron beam N.

Industrial Applicability

According to the invention, it is possible to improve precision of a dose of a neutron beam.

**Claims**

1. A neutron beam irradiation apparatus (1) for irradiating an irradiation object (40) with a neutron beam (N), comprising:

   charged particle beam generating means (10) for generating a charged particle beam (P);
   neutron beam generating means (36) for generating the neutron beam (N) by irradiating a target (T) with the charged particle beam (P);
   **characterized by**:

   measuring means (32, 102) for measuring a dose of the charged particle beam (P) in real time during irradiation with the neutron beam (N) without affecting the charged particle beam (P); and
   a conversion unit (102) for converting the dose of the charged particle beam (P) measured by the measuring means (32, 102) into a dose of the neutron beam (N).

2. The neutron beam irradiation apparatus according to claim 1, further comprising control means (102) for controlling the irradiation of the irradiation object (40) with the neutron beam (N) on the basis of the dose of the charged particle beam (P) measured by the measuring means (32, 102).

3. The neutron beam irradiation apparatus according to claim 1, further comprising display means (104) for displaying the dose of the neutron beam (N) converted by the conversion unit (102).

4. A method of monitoring a neutron beam irradiation apparatus (1) that includes charged particle beam generating means (10) for generating a charged particle beam (P) and neutron beam generating means (36) for generating a neutron beam (N) by irradiating a target (T) with the charged particle beam (P) wherein the neutron beam irradiation apparatus (1) comprises measuring means (32, 102) and a conversion unit (102);
**characterized by**:

the measuring means (32, 102) measuring a dose of the charged particle beam (P) without affecting the charged particle beam (P) and without irradiating an irradiation object (40) with the neutron beam (N), and
the conversion unit (102) converting the dose of the charged particle beam (P) measured by the measuring means (32, 102) into a calculated dose of the neutron beam (N).

5. The control method of a neutron beam irradiation apparatus according to claim 4, wherein the neutron beam irradiation apparatus (1) further comprises display means (104), the display means (104) displaying the calculated dose of the neutron beam (N) converted by the conversion unit (102).

**Patentansprüche**

1. Neutronenstrahlbestrahlungsvorrichtung (1) zum Bestrahlen eines Bestrahlungsobjekts (40) mit einem Neutronenstrahl (N), die Folgendes aufweist:

ein Erzeugungsmittel (10) eines Strahls geladener Teilchen zum Erzeugen eines Strahls (P) geladener Teilchen;
ein Neutronenstrahlerzeugungsmittel (36) zum Erzeugen des Neutronenstrahls (N) durch Bestrahlen eines Ziels (T) mit dem Strahl (P) geladener Teilchen;
**gekennzeichnet durch**:

ein Messmittel (32, 102) zum Messen einer Dosis des Strahls (P) geladener Teilchen, und zwar in Echtzeit während der Bestrahlung mit dem Neutronenstrahl (N) ohne den Strahl (P) geladener Teilchen zu beeinflussen; und
eine Umwandlungseinheit (102) zum Umwandeln der Dosis des Strahls (P) geladener Teilchen, die **durch** das Messmittel (32, 102) gemessen wurde, in eine Dosis des Neutronenstrahls (N).

2. Neutronenstrahlbestrahlungsvorrichtung gemäß Anspruch 1, die ferner ein Steuermittel (102) zum Steuern der Bestrahlung des Bestrahlungsobjekts (40) mit dem Neutronenstrahl (N) basierend auf der Dosis des Strahls (P) geladener Teilchen, die durch das Messmittel (32, 102) gemessen wird, aufweist.

3. Neutronenstrahlbestrahlungsvorrichtung gemäß Anspruch 1, die ferner ein Anzeigemittel (104) zum Anzeigen der Dosis des Neutronenstrahls (N) aufweist, die durch die Umwandlungseinheit (102) umgewandelt wurde.

4. Verfahren zum Überwachen einer Neutronenstrahlbestrahlungsvorrichtung (1), die ein Erzeugungsmittel (10) eines Strahls geladener Teilchen zum Erzeugen eines Strahls (P) geladener Teilchen und ein Neutronenstrahlerzeugungsmittel (36) zum Erzeugen eines Neutronenstrahls (N) durch Bestrahlen eines Ziels (T) mit dem Strahl (P) geladener Teilchen aufweist,
wobei die Neutronenstrahlbestrahlungsvorrichtung (1) ein Messmittel (32, 102) und eine Umwandlungseinheit (102) aufweist;
**dadurch gekennzeichnet, dass**:

das Messmittel (32, 102) eine Dosis des Strahls (P) geladener Teilchen ohne Beeinflussung des Strahls (P) geladener Teilchen und ohne Bestrahlen eines Bestrahlungsobjekts (40) mit dem Neutronenstrahl (N) misst, und
die Umwandlungseinheit (102) die Dosis des Strahls (P) geladener Teilchen, die durch das Messmittel (32, 102) gemessen wird, in eine berechnete Dosis des Neutronenstrahls (N) umwandelt.

5. Steuerverfahren einer Neutronenstrahlbestrahlungsvorrichtung gemäß Anspruch 4, wobei die Neutronenstrahlbestrahlungsvorrichtung (1) ferner ein Anzeigemittel (104) aufweist, wobei das Anzeigemittel (104) die berechnete Dosis des Neutronenstrahls (N) anzeigt, die durch die Umwandlungseinheit (102) umgewandelt wurde.

**Revendications**

1.  Appareil d'irradiation par faisceau de neutrons (1) pour irradier un objet d'irradiation (40) avec un faisceau de neutrons (N), comprenant :

    des moyens de génération de faisceau de particules chargées (10) pour générer un faisceau de particules chargées (P) ;
    des moyens de génération de faisceau de neutrons (36) pour générer le faisceau de neutrons (N) en irradiant une cible (T) avec le faisceau de particules chargées (P) ;
    **caractérisé par** :

    des moyens de mesure (32, 102) pour mesurer une dose du faisceau de particules chargées (P) en temps réel pendant l'irradiation avec le faisceau de neutrons (N)) sans affecter le faisceau de particules chargées (P) ; et
    un module de conversion (102) pour convertir la dose du faisceau de particules chargées (P) mesurée par les moyens de mesure (32, 102) en une dose du faisceau de neutrons (N).

2.  Appareil d'irradiation par faisceau de neutrons selon la revendication 1, comprenant en outre des moyens de commande (102) pour contrôler l'irradiation de l'objet d'irradiation (40) avec le faisceau de neutrons (N) sur la base de la dose du faisceau de particules chargées (P) mesurée par les moyens de mesure (32, 102).

3.  Appareil d'irradiation par faisceau de neutrons selon la revendication 1, comprenant en outre des moyens d'affichage (104) pour afficher la dose du faisceau de neutrons (N) convertie par le module de conversion (102).

4.  Procédé de surveillance d'un appareil d'irradiation par faisceau de neutrons (1) qui comprend des moyens de génération de faisceau de particules chargées (10) pour générer un faisceau de particules chargées (P) et des moyens de génération de faisceau de neutrons (36) pour générer un faisceau de neutrons (N) en irradiant une cible (T) avec le faisceau de particules chargées (P),
    dans lequel l'appareil d'irradiation par faisceau de neutrons (1) comprend des moyens de mesure (32, 102) et un module de conversion (102) ;
    **caractérisé en ce que** :

    les moyens de mesure (32, 102) mesurent une dose du faisceau de particules chargées (P) sans affecter le faisceau de particules chargées (P) et sans irradier un objet d'irradiation (40) avec le faisceau de neutrons (N), et le module de conversion (102) convertit la dose du faisceau de particules chargées (P) mesurée par les moyens de mesure (32, 102) en une dose calculée du faisceau de neutrons (N).

5.  Procédé de commande d'un appareil d'irradiation par faisceau de neutrons selon la revendication 4, dans lequel l'appareil d'irradiation par faisceau de neutrons (1) comprend en outre des moyens d'affichage (104),
    les moyens d'affichage (104) affichant la dose calculée du faisceau de neutrons (N) convertie par le module de conversion (102).

**Fig.1**

EP 2 600 356 B1

*Fig.2*

CURRENT MONITOR

CHARGED PARTICLE BEAM SCANNING UNIT

EP 2 600 356 B1

# Fig.3

CURRENT VALUE OF
CHARGED PARTICLE BEAM

NEUTRON BEAM DOSE RATE

# Fig.4

**EP 2 600 356 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2897502 A
- US 5392319 A
- US 4112306 A
- US 2001014134 A1

**Non-patent literature cited in the description**

- **BLISS M et al.** Real-time dosimetry for boron neutron-capture therapy. *NUCLEAR SCIENCE SYMPOSIUM AND MEDICAL IMAGING CONFERENCE, 1994 IEEE CONFERENCE RECORD,* 30 October 1994, vol. 2, 935-939